# EUROPÄISCHE PATENTSCHRIFT

(11) **EP 0 818 450 B1**
(45) Veröffentlichungstag und Bekanntmachung des Hinweises auf die Patenterteilung: **15.01.2003**
(21) Anmeldenummer: 97810425.5
(22) Anmeldetag: 02.07.1997
(51) Int. Cl.: C07D 251/70, A61K 7/42, C07D 251/50, C07D 251/52

(54) **Triazinderivate als UV-Filter in Sonnenschutzmitteln**
Triazine derivatives as UV filter in sunscreen products
Dérivés de triazine en tant que filtre UV dans des produits antisolaires

(30) Priorität: 08.07.1996 CH 170696
(43) Veröffentlichungstag der Anmeldung: 14.01.1998
(60) Teilanmeldung: 02025811.7
(73) Patentinhaber: Ciba Specialty Chemicals Holding Inc., 4057 Basel (CH)
(72) Erfinder: Metzger, Georges, 68480 Moernach (FR); Reinehr, Dieter, 79400 Kandern (DE); Luther, Helmut, 79639 Grenzach-Wyhlen (DE)

(56) Entgegenhaltungen:
- EP-A- 0 087 098
- EP-A- 0 196 275
- EP-A- 0 202 611
- EP-A- 0 305 190
- EP-A- 0 517 104
- EP-A- 0 570 838
- EP-A- 0 796 851
- DE-B- 1 017 616
- US-A- 4 839 188

## Beschreibung

Die vorliegende Erfindung betrifft die Verwendung s-Triazinverbindungen der Formel 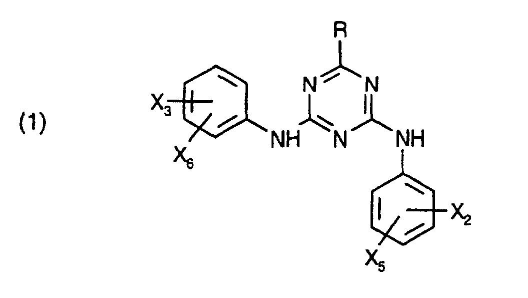 worin
- R: Halogen; geradkettiges oder verzweigtes C₁-C₂₂-Alkyl; geradkettiges oder verzweigtes C₁-C₂₂-Alkoxy; geradkettiges oder verzweigtes C₁-C₂₂-Hydroxyalkoxy; geradkettiges oder

verzweigtes C₁-C₂₂-Hydroxyalkyl; -NHR₁; 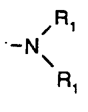 ; oder einen Rest der Formel (1a) 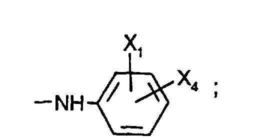 X₁, X₂ und X₃ 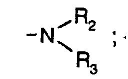
- R₁: Wasserstoff; geradkettiges oder verzweigtes C₁-C₂₂-Alkyl; gegebenenfalls mit einem oder mehreren C₁-C₄-Alkyl substituiertes C₆-C₁₂-Aryl; gegebenenfalls mit einem oder mehreren C₁-C₄-Alkyl substituiertes C₇-C₁₀-Aralkyl; C₅-C₈-Cycloalkyl; oder einen Rest der Formel 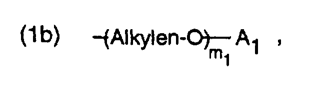
worin
A₁ geradkettiges oder verzweigtes C₁-C₈-Alkyl; C₅-C₈-Cycloalkyl; gegebenenfalls mit einem oder mehreren C₁-C₄-Alkyl substituiertes C₆-C₁₂-Aryl; oder gegebenenfalls mit einem oder mehreren C₁-C₄-Alkyl substituiertes C₇-C₁₀-Aralkyl; und
m₁ 1 bis 10; bedeuten,
R₂ und R₃ unabhängig voneinander Wasserstoff; geradkettiges oder verzweigtes C₁-C₂₂-Alkyl; C₅-C₈-Cycloalkyl; gegebenenfalls mit einem oder mehreren C₁-C₄-Alkyl substituiertes C₆-C₁₂-Aryl; gegebenenfalls mit einem oder mehreren C₁-C₄-Alkyl substituiertes C₇-C₁₀-Aralkyl; einen Rest der Formel (1b); oder einen Rest der Formel 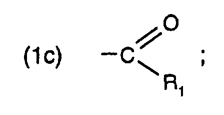
X₄, X₅ und X₆ Wasserstoff; oder Hxdroxy; bedeuten;
als lichtschutzmittel in kosmetischen Präparaten.

Geradkettiges und verzweigtes C₁-C₂₂-Alkyl sind z.B. Methyl, Ethyl, Propyl, Isopropyl, n-Butyl, sec-Butyl, Isobutyl, t-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl, Tridecyl, Tetradecyl, Pentadecyl, Hexadecyl, Heptadecyl, Octadecyl oder Eicosyl.

Beispiele für geradkettiges und verzweigtes C₁-C₁₈-Alkoxy sind z. B. Methoxy, Ethoxy, Propoxy, Isopropoxy, Butoxy, Isobutoxy, sek.-Butoxy, tert.-Butoxy, Pentoxy, Isopentoxy, n-Heptyloxy, n-Octyloxy, Isooctyloxy, n-Nonyloxy, Isononyloxy, Decyloxy, n-Dodecyloxy, Heptadecyloxy Octadecyloxy oder Eicosyloxy.

C₅-C₈-Cycloalkyl bedeutet z.B. Cyclopentyl, Cycloheptyl, Cyclooctyl und insbesondere Cyclohexyl.

Als Beispiele für C₆-C₁₂-Aryl sind insbesondere Phenyl, Naphthyl und Biphenylyl zu nennen.

Beispiele für C₇-C₁₀-Aralkyl sind Benzyl, Phenethyl, α-Methylphenethyl oder α,α-Dimethylbenzyl.

"Alkylen" in der Formel (1b) bedeutet eine bivalente Alkylengruppe mit 2 bis 5, vorzugsweise 2 bis 4 Kohlenstoffatomen. Es handelt sich dabei vorzugsweise um die -CH₂-CH₂-; -CH₂CH₂CH₂-; -CH₂-CH₂-CH₂-CH₂-; 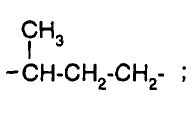 oder 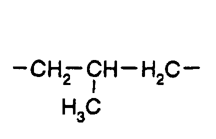 -Gruppe. Unter diesen Alkylen-Gruppen sind ganz besonders die -CH₂-CH₂- und die 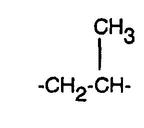 Gruppe bevorzugt.

Halogen bedeutet Fluor, Brom, lod oder vorzugsweise Chlor.

Vorzugsweise kommen Triazinverbindungen der Formel 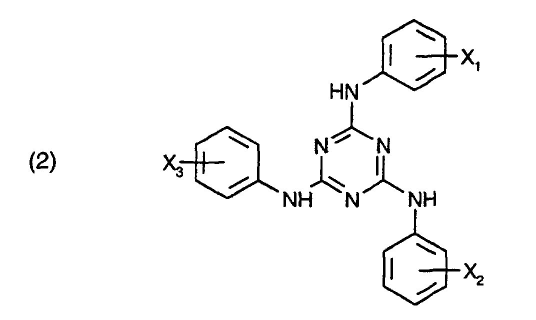 zur Verwendung. X₁, X₂ und X₃ haben dabei die in Formel (1) angegebene Bedeutung.

Im Vordergrund stehen Triazinverbindungen der Formel (1), worin X₁, X₂ und X₃ in ortho-Stellung zum Phenylaminorest des Triazins stehen, also Verbindungen der Formel 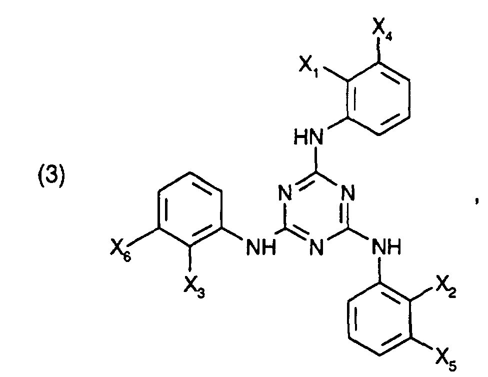 oder ganz besonders Triazinverbindungen der Formel (1), worin X₁, X₂ und X₃ in paraStellung zum Phenylaminorest des Triazins stehen, also Verbindungen der Formel 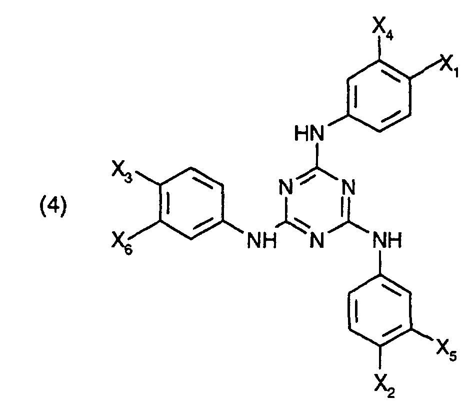 beziehungsweise Verbindungen der Formel

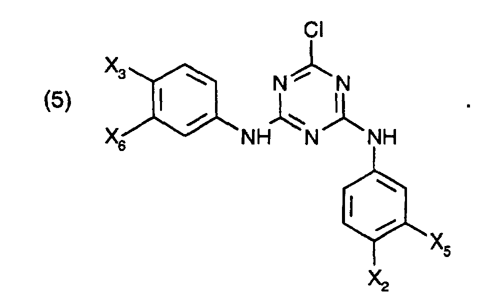

X₁, X₂, X₃, X₄, X₅; und X₆ haben dabei die in Formel (1) angegebene Bedeutung.

Unter den erfindungsgemäss verwendbaren Verbindungen der Formel (1) sind wiederum diejenigen bevorzugt, worin
- R₂: Wasserstoff und
- R₃: einen Rest der Formel 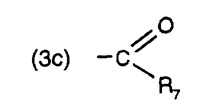 bedeutet, worin
R₇ geradkettiges oder verzweigtes C₁-C₂₂-Alkyl oder einen Rest der Formel (1b) bedeutet.

Insbesondere sind Triazinverbindungen der Formel (1) bevorzugt, worin X₁, X₂ und X₃ die gleiche Bedeutung haben.

Im Vordergrund des Interesses stehen dabei Triazinverbindungen der Formel 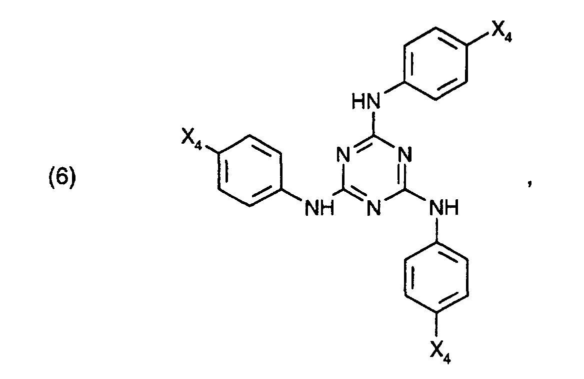 worin
- X₄: 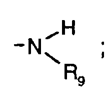
- R₉: einen Rest der Formel (6a) 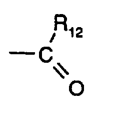
- R₁₂: Wasserstoff; oder geradkettiges oder verzweigtes C₁-C₂₂-Alkyl;
bedeuten.

Ganz besonders interessant sind dabei Verbindungen der Formel (6), worin
- X₄: 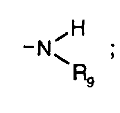
worin
R₉ die oben angegebene Bedeutung hat.

Die Herstellung der erfindungsgemässen Triazinverbindungen der Formel (1) erfolgt in an sich bekannter Weise, wie z.B. durch Umsetzung von 1 Mol Cyanurchlorid mit jeweils 1 Mol der entsprechenden Anilinverbindungen der Formeln (7a) 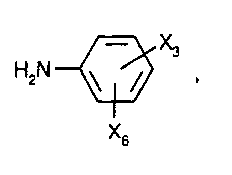 (7b) 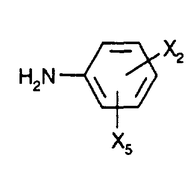 oderr R-H.

R, X₂, X₃ X₅ und X₆ haben dabei die in Formel (1) angegebene Bedeutung

Die Reaktion wird gewöhnlich bei einer Temperatur von 50 bis 200°C in einem geeigneten Lösungsmittel durchgeführt.

Geeignete Lösungsmittel sind dabei zum Beispiel Acetonitril; Ketone, wie z.B. Aceton oder Methylethylketon; Ether, wie z.B. Diethylether, Diisopropylether, Tetrahydrofuran (THF), Dimethylformamid (DMF) oder Dioxan; aliphatische oder aromatische Kohlenwasserstoffe, wie z.B. Pentan, Heptan, Cyclohexan, Benzol, Toluol, Xylol oder Mischungen davon; oder aliphatische Carboxylsäureester wie z.B. Ethylacetat. Für das erfindungsgemässe Herstellungsverfahren wird als Lösungsmittel vorzugsweise Dimethylformamid (DMF) verwendet.

Die allgemeine Reaktion von Trihalogentriazinverbindungen, wie z.B. Cyanurfluorid oder Cyanurchlorid, bei der die drei Halogenatome durch Aminoreste ersetzt werden, sind bekannt und in der technischen Literatur, insbesondere der Fachliteratur, die sich mit Farbstoffen und optischen Aufhellern befasst, ausführlich beschrieben.

Die erfindungsgemäss verwendeten Triazinverbindungen der Formel (1) eignen sich insbesondere als UV-A-Filter,. Diese Verbindungen eignen sich daher als Lichtschutzmittel in kosmetischen, pharmazeutischen und veterinärmedizinischen Präparaten. Die Verbindungen können sowohl gelöst als auch im mikronisierten Zustand verwendet werden.

Einen weiteren Erfindungsgegenstand bildet daher ein kosmetisches Präparat, enthaltend mindestens eine Verbindung der Formel (1), sowie kosmetisch verträgliche Träger- oder Hilfsstoffe.

Für die kosmetische Verwendung haben die erfindungsgemässen Lichtschutzmittel gewöhnlich eine mittlere Partikelgrösse im Bereich von 0,02 bis 2, vorzugsweise 0,05 bis 1,5, und ganz besonders von 0,1 bis 1,0 µ. Die erfindungsgemässen Triazinverbindungen, die gewöhnlich wasserunlöslich sind, können durch übliche Methoden, z.B. Mahlen mit z.B. einer Düsen-, Kugel-, Vibrations- oder Hammermühle auf die gewünschte Partikelgrösse gebracht werden. Vorzugsweise wird das Mahlen in Anwesenheit von 0,1 bis 30, vorzugsweise 0,5 bis 15 Gew. %, bezogen auf die eingesetzte Triazinverbindung, einer Mahlhilfe, wie z.B. eines alkylierten Vinylpyrrolidon-Polymers, eines Vinylpyrrolidon-Vinylacetat-Copolymers, eines Acylglutamates oder insbesondere eines Phospholipids durchgeführt. Das so erhaltene Nanopigment wird in eine übliche Sonnenschutz-Rezeptur eingearbeitet. Die Herstellung von O/W- oder W/O-Emulsionen mit einem oder mehreren Pigmenten, auch in Gegenwart eines oder mehrerer öl- oder wasserlöslicher UV-Absorber, erfolgt nach bekannten Verfahren für die Herstellungen von Sonnenschutz-Emulsionen.

Die kosmetischen Zubereitungen können neben den erfindungsgemässen Triazinverbindungen auch noch einen oder mehrere weitere UV-Schutzstoffe, wie z.B. Benzophenone, p-Methoxyzimtsäureester, Dibenzoylmethanderivate, Benzylidencampherderivate, p-Aminobenzoesäureester, Salicylsäurederivate, Diphenylacrylat-Derivate, Terephthalyden-Dicamphersulfonsäure, Octyltriazone, Phenylbenzimidazol-Sulfonsäure, Menthylanthranilat, TiO₂ (unterschiedlich umhüllt), ZnO, Mica, Benzotriazole oder Vinylgruppen enthaltende Amide oder Zimtsäureamide enthalten. Solche Schutzstoffe sind z.B. in der GB-A-2,286,774 beschrieben oder auch aus Cosmetics & Toiletries (107), 50ff (1992) bekannt.

Die erfindungsgemässe kosmetische Zubereitung enthält z.B. 0,1 bis 15, vorzugsweise 0,5 bis 10 Gew.-%, bezogen auf das Gesamtgewicht der Zusammensetzung, einer erfindungsgemässen Triazinverbindung der Formel (1) oder eines Gemisches aus diesen Triazinverbindungen und einen kosmetisch verträglichen Hilfsstoff.

Die Herstellung der kosmetischen Zusammensetzung kann durch physikalisches Mischen des oder der Triazinverbindungen mit dem Hilfsstoff durch gewöhnliche Methoden, wie z.B. durch einfaches Zusammenrühren der Einzelkomponenten erfolgen.

Die erfindungsgemässe kosmetische Zubereitung kann als Wasser-in-Öl- oder Öl-in-Wasser-Emulsion, als Öl-in-Alkohol-Lotion, als vesikulare Dispersion eines ionischen oder nichtionischen amphiphilen Lipids, als Gel, fester Stift oder als Aerosol-Formulierung formuliert werden.

Als Wasser-in-Öl- oder Öl-in-Wasser-Emulsion enthält der kosmetisch verträgliche Hilfsstoff vorzugsweise 5 bis 50% einer Ölphase, 5 bis 20% eines Emulgators und 30 bis 90% Wasser. Die Ölphase kann dabei irgendein für kosmetische Formulierungen geeignetes Öl enthalten, wie z.B. ein oder mehrere Kohlenwasserstofföle, ein Wachs, ein natürliches Öl, ein Silikonöl, einen Fettsäureester oder einen Fettalkohol. Bevorzugte Mono- oder Polyole sind Ethanol, Isopropanol, Propylenglykol, Hexylenglycol, Glycerin und Sorbitol.

Für die erfindungsgemässen kosmetischen Formulierungen kann jeder konventionell einsetzbare Emulgator verwendet werden, wie z.B. einer oder mehrere ethoxylierte Ester von natürlichen Derivaten, wie z.B. polyethoxylierte Ester von hydrogeniertem Castor-Öl; oder ein Silikonöl-Emulgator wie z.B. Silikonpolyol; eine gegebenenfalls ethoxylierte Fettsäureseife; ein ethoxylierter Fettalkohol; ein gegebenenfalls ethoxylierter Sorbitanester; eine ethoxylierte Fettsäure; oder ein ethoxyliertes Glycerid.

Die kosmetische Formulierung kann auch weitere Komponenten wie z.B. Emollients, Emulsionsstabilisatoren, Haut-Feuchthaltemittel, Hautbräunungsbeschleuniger, Verdickungsmittel wie z.B. Xanthan, Feuchtigkeit-Retentionsmittel wie z.B. Glycerin, Konservierungsmittel, Duft- und Farbstoffe enthalten.

Die erfindungsgemässen kosmetischen Formulierungen zeichnen sich durch eine hohe UV-Absorption aus und bieten daher einen sehr guten Schutz der menschlichen Haut gegen den schädigenden Einfluss von Sonnenlicht.

Die folgenden Beispiele dienen der Veranschaulichung der Erfindung.

Beispiel 1: 45g (0,3 Mol) p-Aminoacetanilid werden in 90ml Dimethylformamid (DMF) bei Raumtemperatur gelöst. Innerhalb von 1 bis 2 Minuten werden 9,2g (0,05 Mol) Cyanurchlorid eingetragen. Die Reaktion verläuft exotherm, wobei die Temperatur auf ca. 60°C ansteigt. Man rührt bei einer Badtemperatur von 80°C im Ölbad weiter und engt bei 100°C am Rotationsverdampfer ein. Der Rückstand wird in 600ml Wasser langsam eingerührt. Dabei fällt die Verbindung der Formel 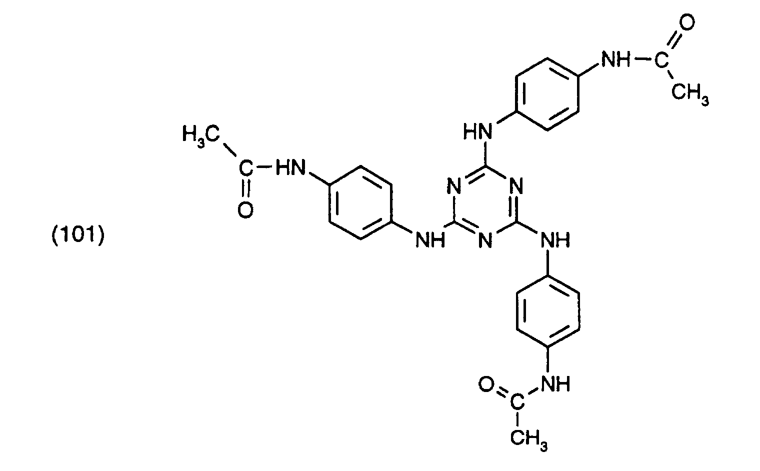 in dicken Flocken aus.

Nach Absaugen, zweimaligem Nachwaschen mit jeweils 30ml Wasser und anschliessendem Verrühren mit Aceton verbleibt ein fast weisses Pulver.
- Ausbeute:: 27,9g (89% d.Th.)
- Fp.:: 215-218°C
- λₘₐₓ:: 292 nm (gemessen in Ethanol)

### Elementaranalyse (berechnet auf C₂₇H₂₇N₉O₃^{·}[4,5 H₂O])

| | C | H | N | O |
|---|---|---|---|---|
| Berechnet: | 51,91 | 5,8 | 20,18 | 22,11 |
| Gefunden: | 51,93 | 5,66 | 20,42 | 21,99 |

Beispiel 2 : Die Verbindung der Formel (101) wird nach Mahlung in Wasser mit Quarzsand und unter Zuhilfenahme von 8% Phospholipid (Phospholipon 80) als Hilfsmittel auf eine mittlere Korngröße von 250 nm gemahlen. Die so erhaltene Nanopigmentsuspension wird in die folgende Rezeptur eingearbeitet:

Zusammensetzung:

| Phase A: | |
|---|---|
| Dimethicone | 2 % |
| Isopropyl Myristate | 9 % |
| Stearylalkohol | 10 % |
| Stearinsäure | 4 % |
| Octyl Methoxycinnamate | 3,5 % |

| Phase B: | |
|---|---|
| Triethanolamin | 1,2 % |
| Carbomer 934 (1%ig) | 5,0 % |
| 50%ige Suspension der Verbindung der Formel (101) | 9,6 % (4,8% Wirkstoff) |
| H₂O | 55,7 % |

Phase A wird separat sehr sorgfältig homogenisiert und ebenso wie Phase B getrennt auf 75-80°C erwärmt. Dann wird Phase B in Phase A unter starkem Rühren zugegeben. Unter Rühren läßt man erkalten.

Der Lichtschutzfaktor dieser Sonnencreme beträgt 15,5 (bestimmt mit dem SPF-Analysator SPF 290 von Optometrics).

Beispiel 3 : Die Verbindung der Formel (101) wird in Wasser mit "Zirkonsand" und unter Hinzufügung von 7% Plantaren 2000 auf eine mittlere Korngröße von 180 nm gemahlen. Die so erhaltene Nanopigmentsuspension wird in folgende Rezeptur eingearbeitet:

### Zusammensetzung:

| Phase A: | |
|---|---|
| Ceteareth-6 (and) Stearyl Alkohol | 2 % |
| Ceteareth-25 | 2 % |
| Cetearyl Alkohol | 5 % |
| Caprylic/Capric Triglyceride | 5 % |
| Cetearyl Octanate | 10 % |
| Vaseline | 5 % |

| Phase B: | |
|---|---|
| Propylenglykol | 3,0 % |
| Carbopol 934 | 0,2 % |
| 50%ige Suspension der Verbindung der Formel (101) | 5,0 % (bezogen auf den Wirkstoffgehalt) |
| H₂O | 57,53 % |

| Phase C: | |
|---|---|
| Triethanolamin | 0,27 % |

Phase A und B werden auf 75-80°C erwärmt. Dann wird Phase B in Phase A unter gründlicher Homogenisierung zugegeben. Dannach folgt Phase C und es wird intensiv nachhomogenisiert.

Der SPF dieser O/W Emulsion beträgt 9,5 (bestimmt mit dem SPF-Analysator 290 von Optometrics).

## Patentansprüche

1. Verwendung der Triazinverbindungen der Formel 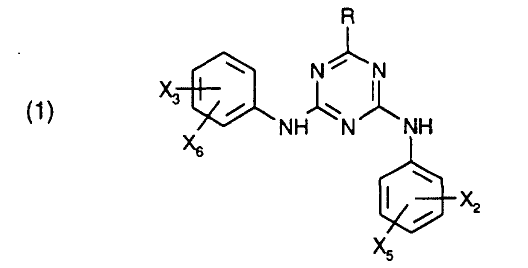 worin
R Halogen; geradkettiges oder verzweigtes C₁-C₂₂-Alkyl; geradkettiges oder verzweigtes C₁-C₂₂- Alkoxy; geradkettiges oder verzweigtes C₁-C₂₂-Hydroxyalkoxy; geradkettiges oder
verzweigtes C₁-C₂₂-Alkoxyalkyl; -NHR₁; 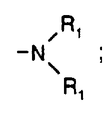 oder einen Rest der Formel (1a) 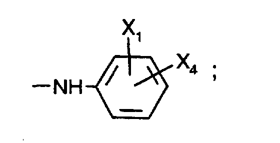 X₁, X₂ und X₃ 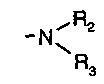
R₁ Wasserstoff; geradkettiges oder verzweigtes C₁-C₂₂-Alkyl; gegebenenfalls mit einem oder mehreren C₁-C₄-Alkyl substituiertes C₆-C₁₂-Aryl; gegebenenfalls mit einem oder mehreren C₁-C₄-Alkyl substituiertes C₇-C₁₀-Aralkyl; C₅-C₈-Cycloalkyl; oder einen Rest der Formel 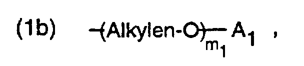
worin
A₁ geradkettiges oder verzweigtes C₁-C₈-Alkyl; C₅-C₈-Cycloalkyl; gegebenenfalls mit einem oder mehreren C₁-C₄-Alkyl substituiertes C₆-C₁₂-Aryl; oder gegebenenfalls mit einem oder mehreren C₁-C₄-Alkyl substituiertes C₇-C₁₀-Aralkyl; und
m₁ 1 bis 10; bedeuten,
R₂ und R₃ unabhängig voneinander Wasserstoff; geradkettiges oder verzweigtes C₁-C₂₂-Alkyl; C₅-C₈-Cycloalkyl; gegebenenfalls mit einem oder mehreren C₁-C₄-Alkyl substituiertes C₆-C₁₂-Aryl; gegebenenfalls mit einem oder mehreren C₁-C₄-Alkyl substituiertes C₇-C₁₀-Aralkyl; einen Rest der Formel (1b); oder einen Rest der Formel 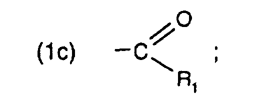
X₄, X₅ und X₆ Wasserstoff; oder Hxdroxy; bedeuten;
als Lichtschutzmittel in kosmetischen Präparaten.

2. Kosmetisches Präparat, enthaltend mindestens eine oder mehrere Verbindungen der Formel (1) nach Anspruch 1 sowie kosmetisch verträgliche Träger- oder Hilfsstoffe, **dadurch gekennzeichnet, dass** es als Wasser-in-Öl- oder Öl-in-Wasser-Emulsion, als Öl-in-Alkohol-Lotion, als vesikulare Dispersion eines ionischen oder nichtionischen amphiphilen Lipids, als Gel, fester Stift oder als Aerosol-Formulierung vorliegt.

3. Präparat nach Anspruch 2, **dadurch gekennzeichnet, dass** es weitere UV-Schutzstoffe enthält.

4. Präparat nach Anspruch 2 oder 3, **dadurch gekennzeichnet, dass** es als weitere UV-Schutzstoffe Benzophenone, p-Methoxyzimtsäureester, Dibenzoylmethanderivate, Benzylidencampherderivate, p-Aminobenzoesäureester, Salicylsäuraederivate, Diphenylacrylat-Derivate, Terephthalyden-Dicamphersulfonsäure, Octyltriazone, Phenylbenzimidazol-Sulfonsäure, Menthylanthranilat, TiO₂ (unterschiedldich umhüllt), ZnO, Mica, Benzotriazole oder Vinylgruppen enthaltende Amide oder Zimtsäureamide enthält.

## Claims

1. The use of a triazine compound of the formula 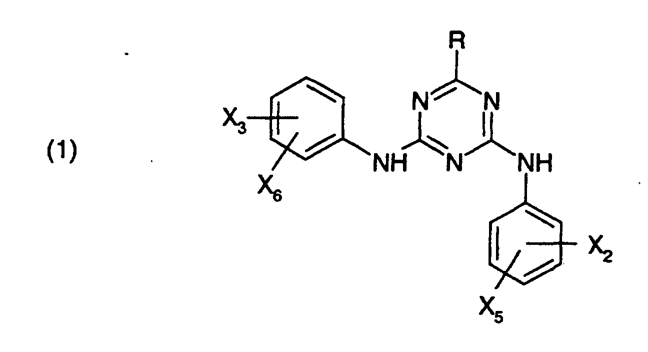 in which
R is halogen; straight-chain or branched C₁-C₂₂alkyl; straight-chain or branched C₁-C₂₂-alkoxy; straight-chain or branched C₁-C₂₂-hydroxyalkoxy; straight-chain or branched C₁-C₂₂alkoxyalkyl; NHR₁; 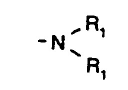 or a radical of the formula(1a) 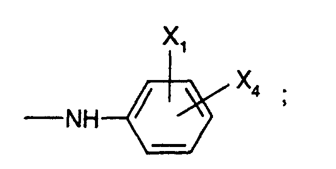 X₁, X₂ and X₃ are 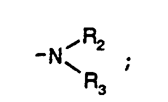
R₁ is hydrogen; straight-chain or branched C₁-C₂₂alkyl; C₆-C₁₂aryl which is unsubstituted, monosubstituted or polysubstituted by C₁-C₄alkyl; C₇-C₁₀aralkyl which is unsubstituted, monosubstituted or polysubstituted by C₁-C₄alkyl; C₅-C₈cycloalkyl; or a radical of the formula 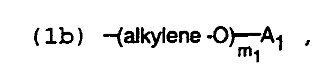 in which
A₁ is straight-chain or branched C₁-C₈alkyl; C₅-C₈-cycloalkyl; C₆-C₁₂aryl which is unsubstituted, monosubstituted or polysubstituted by C₁-C₄alkyl; C₇-C₁₀aralkyl which is unsubstituted, monosubstituted or polysubstituted by C₁-C₄alkyl; and
m₁ is 1 to 10;
R₂ and R₃ independently of one another are hydrogen; straight-chain or branched C₁-C₂₂alkyl; C₅-C₈cycloalkyl; C₆-C₁₂aryl which is unsubstituted, monosubstituted or polysubstituted by C₁-C₄alkyl; C₇-C₁₀aralkyl which is unsubstituted, monosubstituted or polysubstituted by C₁-C₄alkyl; a radical of the formula (1b); or a radical of the formula 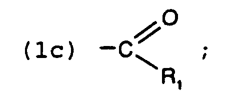
X₄, X₅ and X₆ are hydrogen; or hydroxyl; as sunscreens in cosmetic preparations.

2. A cosmetic preparation, comprising at least one or more compounds of the formula (1) according to claim 1, and cosmetically tolerable carriers or adjuncts, which is in the form of a water-in-oil or oil-in-water emulsion, an oil-in-alcohol lotion, a vesicular dispersion of an ionic or nonionic amphiphilic lipid, a gel, a solid stick or an aerosol formulation.

3. A preparation according to claim 2, which contains further UV protective substances.

4. A preparation according to claim 2 or 3, which as further UV protective substances contains benzophenones, p-methoxycinnamates, dibenzoylmethane derivatives, benzylidene camphor derivatives, p-aminobenzoates, salicylic acid derivatives, diphenyl acrylate derivatives, terephthalydene-dicamphorsulfonic acid, octyltriazones, phenylbenzimidazole-sulfonic acid, menthyl anthranilate, TiO (variously enveloped), ZnO, mica, benzotriazoles or amides containing vinyl groups, or cinnamides.

## Revendications

1. Utilisation des composés triazine de formule 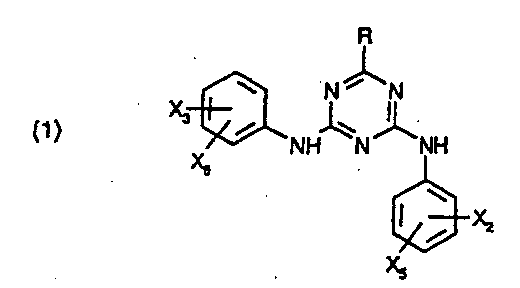 dans laquelle
R représente un halogène ; un groupe alkyle à chaîne linéaire ou ramifiée en C₁ à C₂₂ ; alcoxy à chaîne linéaire ou ramifiée en C₁ à C₂₂ ; hydroxyalcoxy à chaîne linéaire ou ramifiée en C₁ à C₂₂ ; alcoxyalkyle à chaîne linéaire ou ramifiée en C₁ à C₂₂ ; -NH-R₁ ; 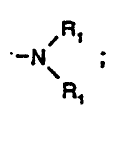 ou un groupe de formule (1a) 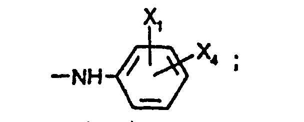
X₁, X₂ et X₃ représentent 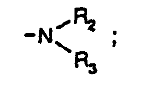
R₁ représente un atome d'hydrogène ; un groupe alkyle à chaîne linéaire ou ramifiée en C₁ à C₂₂ ; aryle en C₆ à C₁₂ éventuellement substitué par un ou plusieurs groupes alkyle en C₁ à C₄ ; aralkyle en C₇ à C₁₀ éventuellement substitué par un ou plusieurs groupes alkyle en C₁ à C₄ ; cycloalkyle en C₅ à C₈ ; ou un groupe de formule
(1b) -(alkylène-O)ₘ₁-A₁,
dans laquelle
A₁ représente un groupe alkyle à chaîne linéaire ou ramifiée en C₁ à C₈ ; cycloalkyle en C₅ à C₈ ; aryle en C₆ à C₁₂ éventuellement substitué par un ou plusieurs groupes alkyle en C₁ à C₄ ; ou aralkyle en C₇ à C₁₀ éventuellement substitué par un ou plusieurs groupes alkyle en C₁ à C₄ ; et
m₁ représente 1 à 10 ;
R₂ et R₃ représentent, indépendamment l'un de l'autre, un atome d'hydrogène ; un groupe alkyle à chaîne linéaire ou ramifiée en C₁ à C₂₂ ; cycloalkyle en C₅ à C₈ ; aryle en C₆ à C₁₂ éventuellement substitué par un ou plusieurs groupes alkyle en C₁ à C₄ ; aralkyle en C₇ à C₁₀ éventuellement substitué par un ou plusieurs groupes alkyle en C₁ à C₄ ; un groupe de formule (1b) ; ou un groupe de formule 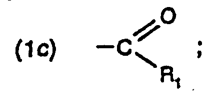
X₄, X₅ et X₆ représentent un atome d'hydrogène ; ou un groupe hydroxy ;
comme produit de protection contre la lumière dans des préparations cosmétiques.

2. Préparation cosmétique, contenant au moins un ou plusieurs composés de formule (1) selon la revendication 1 ainsi que des support ou adjuvants cosmétiquement acceptables, **caractérisée en ce qu'**elle se présente sous forme d'émulsion d'eau dans l'huile ou d'huile dans l'eau, sous forme de lotion d'huile dans l'alcool, sous forme de dispersion vésiculaire d'un lipide amphiphile ionique ou non ionique, sous forme de gel, de crayon solide ou sous forme de formulation d'aérosol.

3. Préparation selon la revendication 2,
**caractérisée en ce qu'**elle contient d'autres substances anti-UV.

4. Préparation selon la revendication 2 ou 3,
**caractérisée en ce qu'**elle contient comme autre substances anti-UV la benzophénone, des esters d'acide p-méthoxycinnamique, des dérivés du dibenzoylméthane, des dérivés de benzilidène-camphre, des esters d'acide p-aminobenzoïque, des dérivés d'acide salicylique, des dérivés d'acrylate de diphényle, l'acide téréphtalidène-dicamphrosulfonique, l'octyltriazone, l'acide phénylbenzimidazole-sulfonique, l'anthranilate de menthyle, TiO₂ (enrobé différemment), ZnO, le mica, des amides contenant du benzotriazole ou des groupes vinyle ou des amides d'acide cinnamique.
